# EUROPEAN PATENT APPLICATION

(11) **EP 3 858 343 A1**
(43) Date of publication of application: **04.08.2021**
(21) Application number: 19864524.4
(22) Date of filing: 18.06.2019
(51) Int. Cl.: A61K 31/20, A23L 33/12, A61P 3/04, A61P 3/06, A61P 43/00

(54) **FAT ACCUMULATION INHIBITOR AND BLOOD LIPID LEVEL IMPROVING AGENT**

(30) Priority: 26.09.2018 JP 2018180696
(71) Applicant: Asahi Group Holdings, Ltd., Tokyo 130-8602 (JP)
(72) Inventor: HAYASHI, Yumeko, Sagamihara-shi, Kanagawa 252-0206 (JP); SUGAWARA, Tomonori, Sagamihara-shi, Kanagawa 252-0206 (JP)
(74) Representative: Vossius & Partner Patentanwälte Rechtsanwälte mbB
(86) International application number: PCT/JP2019/024110
(87) International publication number: WO 2020/066158

(57) **Abstract**

[Problem] To provide a novel technique associated with the inhibition of the accumulation of a fat.

[Solution] A fat accumulation inhibitor comprising 10-hydroxyoctadecanoic acid (10-HOA).

## Description

### Technical Field

The present invention relates to a fat accumulation inhibitor. The present invention also relates to a blood lipid level improving agent.

### Background Art

In recent years, the diet of Japanese people has changed to a great extent. Occasions for high-fat food consumption have increased, and the lack of exercise is advancing. In such a modern lifestyle, the balance between calorie intake and consumption is inclined toward the intake, and as a result, fat is likely to accumulate.

Such accumulation of fat causes obesity, which is an excess of fat accumulation, and significantly damages a healthy and comfortable life. In addition, obesity is an underlying pathological condition of the metabolic syndrome in which the risk of arteriosclerosis such as hypertension, dyslipidemia, and diabetes accumulates.

Therefore, research is being conducted to prevent the accumulation of fat.

For example, research using as an index the activating effect on peroxisome proliferators activated receptor (PPAR) is well known, where PPAR has been identified as a protein that mediates the effect of increasing peroxisomes which are organelles involved in lipolysis. As a result, the effect of 10-hydroxyoctadecanoic acid (10-HOA) on PPAR activation has been reported (Patent Literatures 1 and 2).

### Citation List

### Patent Literature

Patent Literature 1: JP2009-51732
Patent Literature 2: WO 2014/069227

### Summary of Invention

### Technical Problem

The present invention aims to provide a novel technique for inhibiting fat accumulation.

### Solution to Problem

As described above, the effect of 10-HOA on PPAR activation has been disclosed. However, the fact that it has an effect on PPAR activation does not necessarily mean that it inhibits fat accumulation.

The present inventor has earnestly researched and, as a result, has found that 10-HOA has an effect on fat accumulation inhibition. In addition, the present inventor has also found that 10-HOA has an effect on blood lipid level improvement.

The gist of the present invention is as follows.
[1] A fat accumulation inhibitor comprising 10-hydroxyoctadecanoic acid.
[2] The fat accumulation inhibitor according to [1], wherein the fat accumulation inhibitor inhibits accumulation of visceral fat.
[3] The fat accumulation inhibitor according to [1] or [2], wherein the fat accumulation inhibitor inhibits accumulation of perirenal fat and retroperitoneal fat.
[4] A blood lipid level improving agent comprising 10-hydroxyoctadecanoic acid.
[5] The blood lipid level improving agent according to [4], wherein the blood lipid level improving agent reduces a concentration of triglyceride in the blood.
[6] The blood lipid level improving agent according to [4] or [5], wherein the blood lipid level improving agent increases a concentration of small dense high-density lipoprotein cholesterol in the blood.
[7] The blood lipid level improving agent according to any one of [4] to [6], wherein the blood lipid level improving agent inhibits cholesterol absorption in the intestinal tract.
[8] A method for inhibiting fat accumulation, comprising administering 10-hydroxyoctadecanoic acid to a subject.
[9] The method according to [8], wherein the method is for inhibiting accumulation of visceral fat.
[10] The method according to [8] or [9], wherein the method is for inhibiting accumulation of perirenal fat and retroperitoneal fat.
[11] A method for improving the blood lipid level, comprising administering 10-hydroxyoctadecanoic acid to a subject.
[12] The method according to [11], wherein the method is for reducing a concentration of triglyceride in the blood.
[13] The method according to [11] or [12], wherein the method is for increasing a concentration of small dense high-density lipoprotein cholesterol in the blood.
[14] A use of 10-hydroxyoctadecanoic acid in the manufacture of a composition inhibiting fat accumulation.
[15] The use according to [14], wherein the composition inhibits accumulation of visceral fat.
[16] The use according to [14] or [15], wherein the composition inhibits accumulation of perirenal fat and retroperitoneal fat.
[17] A use of 10-hydroxyoctadecanoic acid in the manufacture of a composition improving a blood lipid level.
[18] The use according to [17], wherein the composition reduces a concentration of triglyceride in the blood.
[19] The use according to [17] or [18], wherein the composition increases a concentration of small dense high-density lipoprotein cholesterol in the blood.
[20] The use according to any one of [14] to [19], wherein the composition is a food composition or a pharmaceutical composition.
[21] A non-therapeutic use of 10-hydroxyoctadecanoic acid for inhibiting fat accumulation.
[22] The use according to [21], wherein the use is for inhibiting accumulation of visceral fat.
[23] The use according to [21] or [22], wherein the use is for inhibiting accumulation of perirenal fat and retroperitoneal fat.
[24] A non-therapeutic use of 10-hydroxyoctadecanoic acid for improving a blood lipid level.
[25] The use according to [24], wherein the use is for reducing a concentration of triglyceride in the blood.
[26] The use according to [24] or [25], wherein the use is for increasing a concentration of small dense high-density lipoprotein cholesterol in the blood.

### Advantageous Effects of Invention

According to the present invention, it is possible to provide a novel technique for inhibiting fat accumulation.

### Brief Description of Drawings

[Figure 1] Figure 1 is a graph showing the amount of visceral fat in mice according to the Example.
[Figure 2] Figure 2 is a graph showing the amount of subcutaneous fat in mice according to the Example.
[Figure 3] Figure 3 is a graph showing the total amount of fat in mice according to the Example.
[Figure 4] Figure 4 is a graph showing the concentration of triglyceride in the blood of mice according to the Example.
[Figure 5] Figure 5 is a graph showing the concentration of small dense high-density lipoprotein (HDL) cholesterol in the blood of mice according to the Example.
[Figure 6] Figure 6 is a graph showing the concentration of apolipoprotein A-I in the blood of mice according to the Example.
[Figure 7] Figure 7 is a graph showing the concentration of chylomicron cholesterol in the blood of mice according to the Example.
[Figure 8] Figure 8 is a graph showing the gene expression level of a cholesterol transporter (NPC1L1) in the jejunal epithelial tissue of mice according to the Example.

### Description of Embodiments

### [First Embodiment]

The following describes in detail a first embodiment of the present invention.

The first embodiment relates to a fat accumulation inhibitor, which contains 10-hydroxyoctadecanoic acid (10-HOA) or a salt thereof as an active ingredient. The fat accumulation inhibitor in the first embodiment inhibits the accumulation of visceral fat in particular, and above all, inhibits the accumulation of perirenal fat and retroperitoneal fat.

In the present description, visceral fat refers to adipose tissue accumulated in the abdominal cavity.

In addition, perirenal fat refers to adipose tissue covering the kidneys and the adrenal glands, and retroperitoneal fat refers to adipose tissue accumulated around the abdominal wall on the dorsal side in the abdominal cavity.

10-HOA is also referred to as 10-hydroxystearic acid, and is a saturated hydroxy fatty acid having 18 carbon atoms and having a hydroxyl group at the 10-position.

The 10-HOA to be contained may be organochemically synthesized or derived from a natural product.

The synthesis method for synthesizing 10-HOA is not particularly limited, and 10-HOA can be synthesized, for example, by the method described in Patent Literature 2 mentioned above.

In addition, 10-HOA may be derived from a natural product. The natural product may be made to produce 10-HOA, and the method is not particularly limited. For example, 10-HOA is contained in various lactic acid bacteria (Kishino et al., Polyunsaturated fatty acid saturation by gut lactic acid bacteria affecting host lipid composition, Proc Natl Acad Sci USA October 29, 2013 110 (44) 17808-17813), and therefore, the 10-HOA to be contained may be derived from lactic acid bacteria.

In addition, the fat accumulation inhibitor in the first embodiment may contain a salt of 10-HOA instead of 10-HOA or together with 10-HOA. Examples of such salt include alkali metal and alkaline earth metal salts such as sodium salts, potassium salts, and calcium salts, and acid addition salts.

The fat accumulation inhibitor in the first embodiment may contain other components in addition to 10-HOA or a salt thereof, as long as the object of the present invention can be achieved.

The form (dosage form) of the fat accumulation inhibitor in the first embodiment is not particularly limited, and it can be produced, for example, as a drug, quasi drug, food, drink, or the like for humans.

When the fat accumulation inhibitor in the first embodiment is used as a drug, quasi drug, food or drink, it also can be formulated by appropriately mixing 10-HOA or a salt thereof with, for example, an excipient, a binder, a stabilizer, a disintegrant, a lubricant, a flavoring agent, a suspending agent, a coating agent, and other optional components.

The dosage form can be, for example, tablets, pills, capsules, granules, powders, powder agents, syrups or the like, and it is desirable to administer these orally.

Alternatively, when the fat accumulation inhibitor in the first embodiment is produced in the form of a food or drink, in addition to ordinary foods or drinks, it may be, but is not particularly limited to, a food for special dietary uses, a food with health claims such as a food for specified health uses or a food with nutrient function claims, a food with functional claims, or the like. Specific examples of foods and drinks include dietary supplements (supplements), milk, processed milk, milk drinks, soft drinks, alcoholic beverages, fermented foods and drinks, fermented milk, yogurt, cheese, bread, biscuits, crackers, pizza crust, ice cream, candy, gummies, gums, chocolate, formula milk, liquid foods, foods for medical uses, powdered formulas and foods for infants, powdered formulas and foods for lactating women, freeze-dried foods, seasonings, sauces, noodles, and the like.

In addition, the fat accumulation inhibitor in the first embodiment may be a drug, quasi drug, food, or drink containing a composition containing 10-HOA or a salt thereof.

In addition, the fat accumulation inhibitor in the first embodiment is not limited to drugs, quasi drugs, foods and drinks for humans, and may be in the form of drugs or feeds for non-human animals. Examples of non-human animals include non-human higher vertebrates, particularly non-human mammals, and more specifically, pets such as dogs and cats, and livestock animals such as cows, horses, pigs, and sheep.

The daily intake of the fat accumulation inhibitor in the first embodiment is also not limited particularly. For example, in the case of an adult, the content of 10-HOA or a salt thereof (the total amount when both are contained) may be adjusted so that 0.01 to 100 mg, preferably 0.1 to 10 mg, and more preferably 1 to 6 mg can be ingested per day. The content ratio of 10-HOA or a salt thereof in the fat accumulation inhibitor in the first embodiment is also not limited particularly, and may be appropriately adjusted according to the ease of production, a preferred daily dose, or the like.

As described above, according to the first embodiment, it is possible to provide a novel technique for inhibiting fat accumulation.

To sum up, ingesting 10-HOA or a salt thereof according to the first embodiment in the form of, for example, a drug, quasi drug, food, drink, or the like containing the 10-HOA or a salt thereof as described above allows to inhibit the accumulation of visceral fat and the like (particularly perirenal fat and retroperitoneal fat) in humans or non-human animals having ingested it, albeit with variation between individuals, and as a result, to inhibit fat accumulation, and can be expected to prevent or improve obesity (eliminate or reduce excessive fat accumulation), for example.

### [Second Embodiment]

The following describes in detail a second embodiment of the present invention. The description of the parts common to the first embodiment will be omitted.

The second embodiment relates to a blood lipid level improving agent, which contains 10-HOA or a salt thereof as an active ingredient, in the same way as the fat accumulation inhibitor in the first embodiment.

Here, in the present description, blood lipid level improvement means that the blood lipid level, which may cause arteriosclerosis and the like, is brought to a level at which the symptoms are less likely to occur.

Specific effects include inhibition of the elevation of blood lipid level, reduction of excess lipids in the blood, and maintenance or increase of the lipids that are preventive factors for diseases. In particular, examples of the effects of the blood lipid level improving agent in the second embodiment include the reduction of the triglyceride concentration in the blood, the inhibition of the absorption of cholesterol in the intestinal tract, and the increase in the concentration of small dense high-density lipoprotein (HDL) cholesterol in the blood.

Furthermore, in the present description, a triglyceride is an ester of a fatty acid and glycerin, and specific examples include monoglyceride, diglyceride and triglyceride.

Cholesterol refers to (3β)-cholest-5-en-3-ol, cholest-5-en-3β-ol, its derivatives and analogs thereof, which are described in the International Publication WO 2013/061969.

In the present invention, small HDL cholesterol refers to cholesterol in the HDL having the first to third smallest particle size when HDL is classified into 7 subclasses by particle size (Okazaki M., Bunseki Reprint, "Analysis of Serum Lipoproteins by High Performance Gel Filtration Chromatography" (2000), very small HDL to small HDL). Small HDL usually has a low lipid content among HDLs.

The blood lipid level improving agent in the second embodiment can also have the same composition as the fat accumulation inhibitor in the first embodiment. Moreover, the daily intake of the blood lipid level improving agent in the second embodiment can be the same as that of the fat accumulation inhibitor in the first embodiment.

As described above, according to the second embodiment, it is possible to provide a novel technique for blood lipid level improvement.

To sum up, ingesting 10-HOA or a salt thereof according to the second embodiment in the form of the above-mentioned drug, quasi drug, food, drink, or the like, for example, allows to improve the blood lipid level by the effects of reducing the triglyceride concentration in the blood, inhibiting the absorption of cholesterol in the intestinal tract, increasing the concentration of small HDL cholesterol in the blood, and the like in humans or non-human animals having ingested it, albeit with variation between individuals. As a result, it is possible to reduce the occurrence of arteriosclerosis and the like, which can be expected to contribute to the prevention and improvement of diseases such as heart diseases, cerebrovascular disorders, and the like.

### Example

Hereinafter, the present invention is further described in detail with an Example. However, the present invention is not limited thereto.

The animal experiment was approved by the Animal Experiment Committee of Asahi Group Holdings, Ltd. (March 9, 2017), and was conducted in accordance with the guidelines for the management and use of laboratory animals of the company's R&D center (implementation period: April 3, 2017 to January 10, 2018). Four-week-old C57BL/6N male mice (Japan SLC, Inc.) were purchased and used for the experiment. The mice were raised in the following environment: room temperature: 23±1.5°C, humidity: 55±15%, lighting time: 8:00 to 20:00.

High-fat diet containing 45 kcal% fat (Research Diets, Inc., D12451) was used to prepare feed with or without 0.1% 10-HOA. The four-week-old mice were acclimated to the high-fat diet for 2 weeks, then divided into two groups: (1) high-fat diet control group and (2) 0.1% 10-HOA-containing high-fat diet group, with 10 mice per group, and were administered the diet for 15 weeks. Feed and drinking water (tap water) were given freely by individual housing.

After fasting for 16 hours from the day before dissection, the amount of abdominal visceral fat, subcutaneous fat, and total fat (sum of the amount of visceral fat and subcutaneous fat) were measured by a CT scanner (Latheta LCT-100; Hitachi Aloka Medical, Ltd.), under anesthesia by isoflurane inhalation. Then, the abdomen was opened under anesthesia, and blood was collected from the abdominal vena cava. Visceral fat (perirenal fat, and retroperitoneal fat) and jejunal epithelial tissue were collected to measure the weight of adipose tissue. The above test was repeated three times, and an analysis was performed by a two-way analysis of variance (factor 1: test group, factor 2: number of animal experiments) (n=10×3/group).

The triglyceride concentration in the plasma was measured using a Triglyceride E test. The amount of cholesterol in the plasma was measured using a conventional method (G. Toshima et al., LipoSEARCH (registered trademark); Analytical GP-HPLC method for lipoprotein profiling and its applications, J. Biol. Macromol., 13 (2), 21-32 (2013)) at Skylight Biotech Inc,. Mouse Apolipoprotein A-I ELISAPRO kit (MABTECH) was used for the measurement of apolipoprotein A-I in the plasma. The jejunal epithelial tissue was collected by QIAzol Lysis Reagent (QIAGEN), and total RNA was extracted according to the product protocol of RNeasy Mini Kit (QIAGEN). Total RNA was reverse transcribed by M-MLV Reverse Transcriptase (Life Technologies Japan Ltd.) to prepare cDNA. To measure the mRNA expression level, real-time PCR by the intercalator method was performed using Light Cycler System (Roche Diagnostics K.K.). Thunderbird (registered trademark) SYBR qPCR Mix (TOYOBO CO., LTD.) was used as a PCR enzyme and prepared according to the protocol of the product. 5'-TCCTTCTTCCAGGCTTTGGG-3' (Sequence Listing SEQ ID NO: 1) and 5'-GACACCCTCCAGAAAGCGAG-3' (Sequence Listing SEQ ID NO: 2) were used as primers for 36b4 (internal standard), and 5'-GAGAGCCAAAGATGCTACTATCTTCA-3' (Sequence Listing SEQ ID NO: 3) and 5'-CCCGGGAAGTTGGTCATG-3' (Sequence Listing SEQ ID NO: 4) were used as primers for Npc111. The mRNA expression level was expressed as a relative value to the control group.

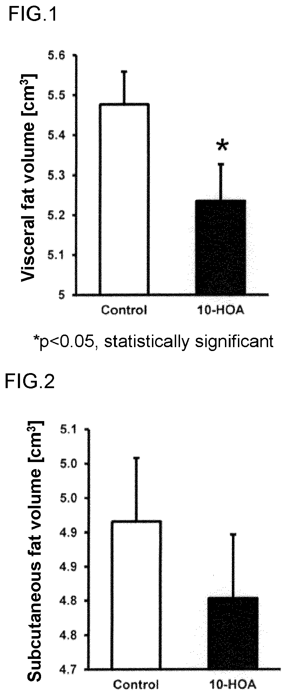

The amount of visceral fat, subcutaneous fat, and total fat of the 10-HOA group and the control group are shown in Figures 1, 2 and 3.

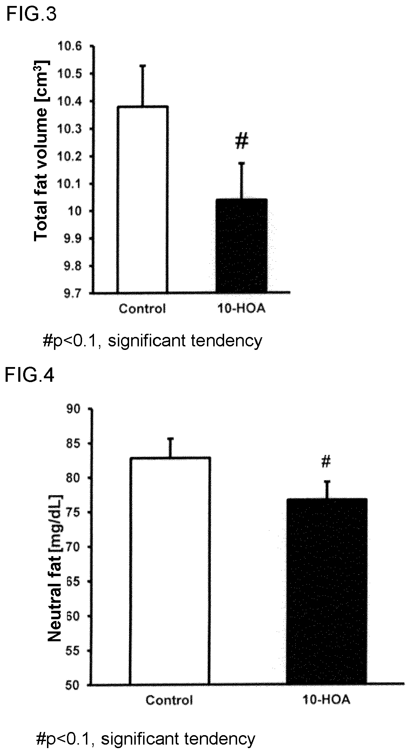

As a result of the administration for 15 weeks, the amount of abdominal visceral fat was significantly reduced in the 10-HOA group compared to the control group (Figure 1), and the total amount of fat showed a decreasing tendency (Figure 3). In addition, among the visceral fats, the weights of perirenal fat and retroperitoneal fat were reduced in the 10-HOA group (Table 1).

**[Table 1]**

| | Control | 10-HOA | |
|---|---|---|---|
| Perirenal fat (g) | 0.5454 ± 0.0166 | 04924 ± 0.0130 | * |
| Fat on the posterior abdominal wall (g) | 0.7608 ± 0.0239 | 0.6805 ± 0.0206 | * |

| | | | |
|---|---|---|---|
| * p<0.05, statistically significant | | | |

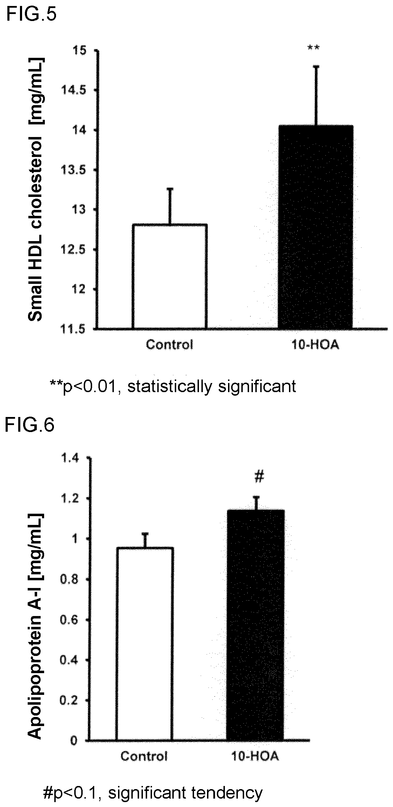

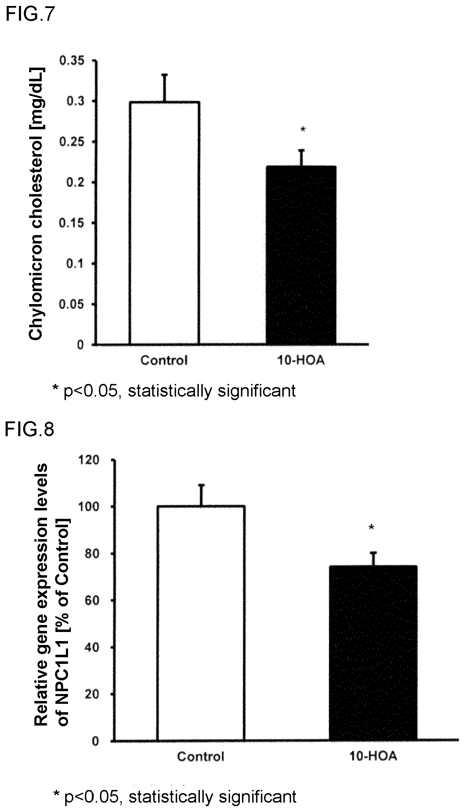

For plasma lipids, a decreasing tendency in the triglyceride concentration was observed (Figure 4), and among HDL cholesterol, small HDL cholesterol, which has a high capability for reverse transporting cholesterol from tissues and is important for reducing the risk of heart disease, was significantly increased (Figure 5). Apolipoprotein A-I, which is a constituent protein of HDL, also showed an increasing tendency (Figure 6), suggesting the possibility of an improvement in the blood lipid metabolism. Furthermore, a significant decrease in chylomicron cholesterol (Figure 7) and a decrease in the gene expression level of the cholesterol transporter (NPC1L1) in jejunal epithelial tissue (Figure 8) were observed, suggesting the inhibition of cholesterol absorption from the small intestine.

The above results showed the inhibitory effect of 10-HOA administration on fat accumulation, suggesting that 10-HOA may improve lipid metabolism in mice organism.

## Claims

1. A fat accumulation inhibitor comprising 10-hydroxyoctadecanoic acid.

2. The fat accumulation inhibitor according to claim 1, wherein the fat accumulation inhibitor inhibits accumulation of visceral fat.

3. The fat accumulation inhibitor according to claim 1 or 2, wherein the fat accumulation inhibitor inhibits accumulation of perirenal fat and retroperitoneal fat.

4. A blood lipid level improving agent comprising 10-hydroxyoctadecanoic acid.

5. The blood lipid level improving agent according to claim 4, wherein the blood lipid level improving agent reduces a concentration of triglyceride in the blood.

6. The blood lipid level improving agent according to claim 4 or 5, wherein the blood lipid level improving agent increases a concentration of small dense high-density lipoprotein cholesterol in the blood.

7. The blood lipid level improving agent according to any one of claims 4 to 6, wherein the blood lipid level improving agent inhibits cholesterol absorption in the intestinal tract.

8. A method for inhibiting fat accumulation, comprising administering 10-hydroxyoctadecanoic acid to a subject.

9. A method for improving a blood lipid level, comprising administering 10-hydroxyoctadecanoic acid to a subject.


SEQUENCE LISTING

<110>  Asahi group holdings, ltd.

<120>  An agent for inhibiting fat accumulation and an agent for
       improving  lipids in blood

<130>  FP0752PCT

<160>  4

<170>  PatentIn version 3.5

<210>  1
<211>  20
<212>  DNA
<213>  artificial  sequence

<220>
<223>  36b4 primer

<400>  1
tccttcttcc aggctttggg                                                   20


<210>  2
<211>  20
<212>  DNA
<213>  artificial sequence

<220>
<223>  36b4 primer

<400>  2
gacaccctcc agaaagcgag                                                   20


<210>  3
<211>  26
<212>  DNA
<213>  artificial sequence

<220>
<223>  Npc1l1 primer

<400>  3
gagagccaaa gatgctacta tcttca                                            26


<210>  4
<211>  18
<212>  DNA
<213>  artificial sequence

<220>
<223>  Npc1l1 primer

<400>  4
cccgggaagt tggtcatg                                                     18